# EUROPEAN PATENT APPLICATION

(11) **EP 3 679 910 A1**
(43) Date of publication of application: **15.07.2020**
(21) Application number: 18854671.7
(22) Date of filing: 10.09.2018
(51) Int. Cl.: A61F 13/02, A61F 13/00

(54) **TENSION-ATTENUATING HYPERTROPHIC SCAR REDUCING FILM**

(30) Priority: 08.09.2017 KR 20170115355
(71) Applicant: Yi, Jeong Yoon, Daejeon 34032 (KR); Koo, Bon-Aa, Daejeon 34032 (KR); Yi, Su Ho, Daejeon 34032 (KR); Yi, Hwa Jeong, Daejeon 34032 (KR)
(72) Inventor: Yi, Jeong Yoon, Daejeon 34032 (KR); Koo, Bon-Aa, Daejeon 34032 (KR); Yi, Su Ho, Daejeon 34032 (KR); Yi, Hwa Jeong, Daejeon 34032 (KR)
(74) Representative: btb IP Bungartz Baltzer Partnerschaft mbB Patentanwälte
(86) International application number: PCT/KR2018/010537
(87) International publication number: WO 2019/050354

(57) **Abstract**

The present invention relates to a tension attenuating hypertrophic scar reduction film, and more particularly, the tension attenuating hypertrophic scar reduction film that prevents the opening of the wound by tension by distributing or removing the tension transmitted to the wound by attaching the film of different thickness or asymmetric size to the wound where the tension is applied.

## Description

### CROSS-REFERENCE TO RELATED APPLICATIONS

This application claims the benefit under 35 U.S.C. section 371, of PCT International Application No.: PCT/KR2018/010537, filed on September 10, 2018, which claims foreign priority to Korean Patent Application No.: 10-2017-0115355, filed on September 8, 2017, in the Korean Intellectual Property Office, both of which are hereby incorporated by reference in their entireties.

### TECHNICAL FIELD

The present invention relates to a tension attenuating hypertrophic scar reduction film, and more particularly, the tension attenuating hypertrophic scar reduction film that prevents the opening of the wound by tension by distributing or removing the tension transmitted to the wound by attaching the film of different thickness or asymmetric size to the wound where the tension is applied.

### BACKGROUND TECHOLOGY

A hypertrophic scar is a trauma including burns or a type of scar that appears on the skin after damaged to the skin by a surgery. This means that the scars bounce off due to tension on the damaged skin and grow wide.

That is, when the skin is damaged, the damaged skin is removed as the inflammation occurs, and the damaged skin is healed as the cell proliferation is performed. At this time, the proliferation of the cells varies depending on the size of the trauma, and if additional tension is generated on the trauma, the generated tension widens the interval of the trauma, thereby increasing the cell proliferation range, When the cell proliferation is excessively formed on the trauma area and the tension is removed, the overproliferated cells protrude outward from the skin surface, causing cosmetic problems.

Conventional methods of treating thickening scars include applying an ointment continuously, removing scars by incision, or compressing by compression stocking so that the scars do not bounce.

The application of the ointment is possible in the case of a relatively small trauma. In the case of a cut, a wound by an edged weapon, a gash, a tension is applied to the wound site depending on the formation position, which is not suitable for reducing hypertrophic scar formation.

In addition, an incision is also a different method from minimizing scar generation beforehand in a post-mortem manner that is added after trauma.

In addition, in the case of compression stocking is mainly used in the wound area by surgery, it has the effect of reducing the scar to some extent by limiting movement by compressing relatively wide area including wound site, by limiting the degree of expansion can reduce the tension transmitted to the wound site where the expansion and contraction is repeated, such as the abdomen. This creates a significant repulsion when the wearer breathe or the stomach is swollen by taking food.

Korean Utility Model Registration No. 20-0288594 (registered on Aug. 29, 2002; hereafter referred to as "cited document") suggested a band-aid to prevent the opening of wounds.

The cited document is a band-aid made of a material that can be stretched only in one direction, a water-soluble antibiotics applied to the outside of the band-aid to protect and sterilize the wound site, and melt by the blood from the wound site to restore the elasticity of the band-aid; and an adhesive configured to attach the band-aid to the wound to prevent the wound from being opening.

However, since the prior document does not consider the difference in the magnitude of the tension acting on both sides of the wound formed in the longitudinal direction, a strong tension is transmitted to any one side of the band-aid by non-uniform tension, the band-aid is unevenly transmitted tension can cause distortion, the deformation of the band-aid is also transmitted to the tension of the tissue attached to it, thereby scarring may occur due to open wounds or non-uniform tension.

Therefore, even if non-uniform tension occurs on both sides of the wound site, there is a need for a new way of reducing scars that can reduce scarring by attenuated the tension on the wound.

### DETAILED DESCRIPTION OF THE INVENTION

### TECHNICAL PROBLEMS

The present invention has been made to solve the conventional problems as described above, the object of the present invention, provided in a form of a film configured to be attached to the wound site, it is an object of the present invention to provide a tension attenuating hypertrophic scar reduction film which redistributes or reduces the tension acting on the wound site thereby minimizing the tissue tension of the wound site.

Means for solving the problems:
In order to achieve this object, a tension attenuating hypertrophic scar reduction film may include: a film body made of a synthetic resin and having a non-uniform thickness; and an adhesive part coated on the film body so that the film body is attached to the wound site.

According to one example, the film main body is formed gradually reduced in thickness away from the wound site, i.e., as the distance from the wound site increases, a thickness of the film main body gradually decreases.

In addition, an embodiment of the present invention, a tension attenuating hypertrophic scar reduction film may include: a film main body made of synthetic resin; and an adhesive part coated on the film body so that the film body is attached to the wound site, wherein both sides of the film main body are formed asymmetrically in one or more of the length and area with respect to the wound site.

According to one example, the film main body is asymmetrically formed in both sides of the film main body about the wound site, wherein a relatively long side of the two sides is attached to a relatively strong tension side of the wound site and a relatively short side of the two sides is attached to a relatively weak tension side of the wound site. According to an embodiment of the present invention, the film main body is asymmetrically formed to have both sides of areas about the wound site, wherein a relatively large area side is attached to a relatively strong tension side of the wound site and a relatively small area side is attached to a relatively weak tension side of the wound site.

A tension attenuating hypertrophic scar reduction film may include: a film main body configured to be folded about a folding line and attached to a wound site; and an adhesive part coated on one side or both sides of the film main body configured to be attached to the wound site according to an aspect of the present invention.

According to one aspect of the present invention, example, the film main body may include: the first film main body attached to a wound site; and a second film main body extending from an end of the first film main body and folded in a direction in which the first film main body is positioned using a first folding line to overlap the first film main body.

### EFFECTS OF THE INVENTION

According to an embodiment of the present invention, by dispersing a tension generated on the wound site to the outside of the wound site, the tension transmitted to the wound site can be reduced, thereby suppressing the hypertrophic scar formation caused by a wound opening, i.e., a wound dehiscence.

In particular, a large area of film is attached to the human body where a strong tension is generated so that the tension is transmitted to the film through the large area, the received tension is attenuated by elastic deformation in the film. The attenuated tension is redistributed to a distant part of the wound site so that substantially minimize the tension transferring to the wound site, thereby providing an effect of suppressing the scarring caused by the wound opening through two stages of fixing the wound site and attenuating the tension.

Effects of the present invention are not limited to the above-mentioned effects, and other effects not mentioned will be clearly understood by those skilled in the art from the description of the claims.

### BRIEF DESCRIPTIONS OF THE DRAWINGS

- FIG. 1: is a perspective view and a side view of a tension attenuating hypertrophic scar reduction film according to a first embodiment of the present invention.
- Fig. 2: is another perspective view and a side view of a tension attenuating hypertrophic scar reduction film according to a first embodiment of the present invention.
- FIG. 3: is a top and side views of an attenuating hypertrophic scar reduction film according to a second embodiment of the present invention.
- Fig. 4: is a plan view of a tension attenuating hypertrophic scar reduction film according to a third embodiment of the present invention.
- FIG. 5: is a view showing the top and the bottom sides of a tension attenuating hypertrophic scar reduction film according to a fourth embodiment of the present invention.
- FIG. 6: is a side view illustrating a folded state of an attenuating hypertrophic scar reduction film of FIG. 5 according to a fourth embodiment of the present invention.
- FIG. 7: is top and bottom side views of a tension attenuating hypertrophic scar reduction film according to the fifth embodiment of the present invention.
- FIG. 8: is a side view illustrating a folded state of the tension attenuating hypertrophic scar reduction film of FIG. 7.
- FIG. 9: is a view showing the top and bottom sides of a tension attenuating hypertrophic scar reduction film according to a sixth embodiment of the present invention.

### [EXPLANATIONS OF NUMERIC REFERENCES]

| | | | |
|---|---|---|---|
| 10, 30, 40, 50, 70, 90: | Tension attenuating hypertrophic scar reduction film | | |
| 11: | Film main body | 13: | Adhesive Part |
| 15: | Variation Prevention Part 1 | 7: | Variable elastic Part |
| 31: | Film main body | 33: | Adhesive Part |
| 35: | Long film part | 37: | short film part |
| 41: | film main body | 45: | first film part |
| 47: | Second film part | 51: | First folding line |
| 53: | Film main body | 54: | Close contact part |
| 55: | Adhesive part | 56: | firsts adhesive part |
| 57: | first film main body | 58: | second adhesive part |
| 59: | second film main body | 61: | non-adhesive part |
| 63: | Bent part | 71: | Film part |
| 72: | Adhesive part | 73: | First film part |
| 74: | First folding line | 75: | Second film part |
| 76: | Second folding line | 77: | Third film part |
| 78: | First adhesive part | 79: | Second adhesive part |
| 81: | third adhesive part | 83: | non-adhesive part |
| 85: | non-adhesive part | 87: | first bent part |
| 89: | Second bent part | 91: | First film part |
| 92: | film part | 93: | folding line |
| 94: | adhesive part | 95: | a second main film part |
| 97: | first adhesive part | 99: | first adhesive part |

### EMBODIMENTS FOR INVENTION

Hereinafter, some embodiments of the present invention will be described in detail through exemplary drawings. In adding reference numerals to the components of each drawing, it should be noted that the same reference numerals are assigned to the same components as much as possible even though they are shown in different drawings. In describing the present invention, when it is determined that the detailed description of the related well-known configuration or function may obscure the gist of the present invention, the detailed description thereof will be omitted.

In addition, in describing the component of this invention, terms, such as 1st, 2nd, A, B, (a), (b), can be used. These terms are only for distinguishing the components from other components, and the nature, order or order of the components are not limited by the terms. If a component is listed as being "connected", "coupled" or "connected" to another component, the component may be directly connected to or connected to the other component, it is to be understood that another component may be "connected", "coupled" or "connected" between each component.

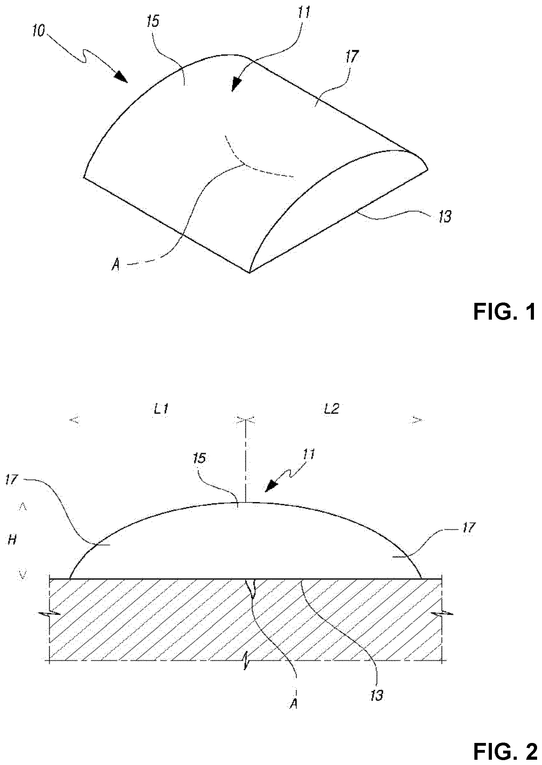

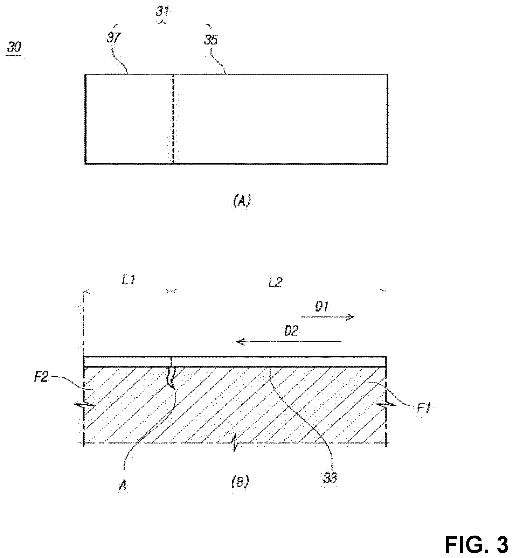

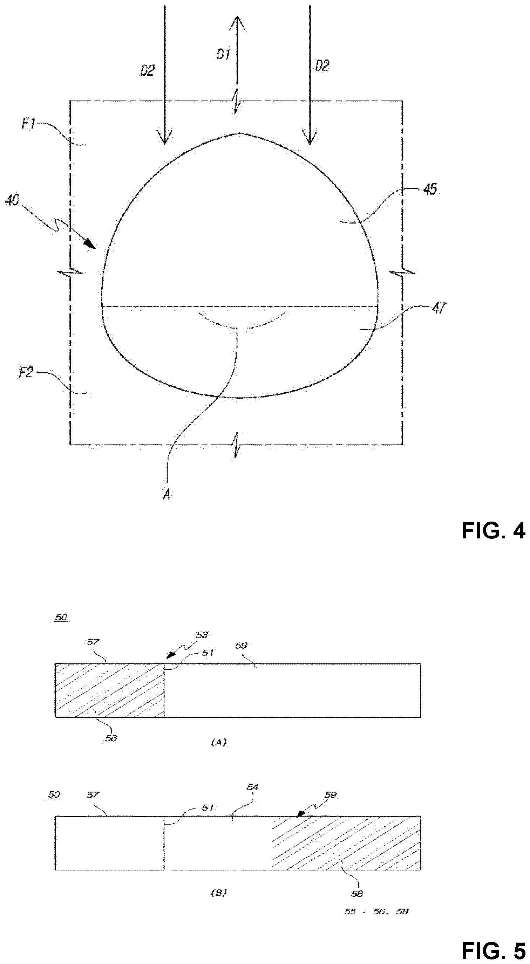

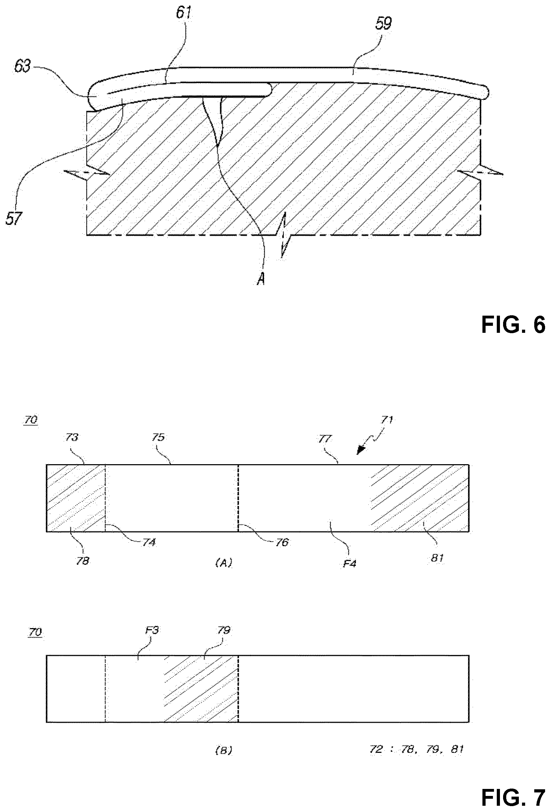

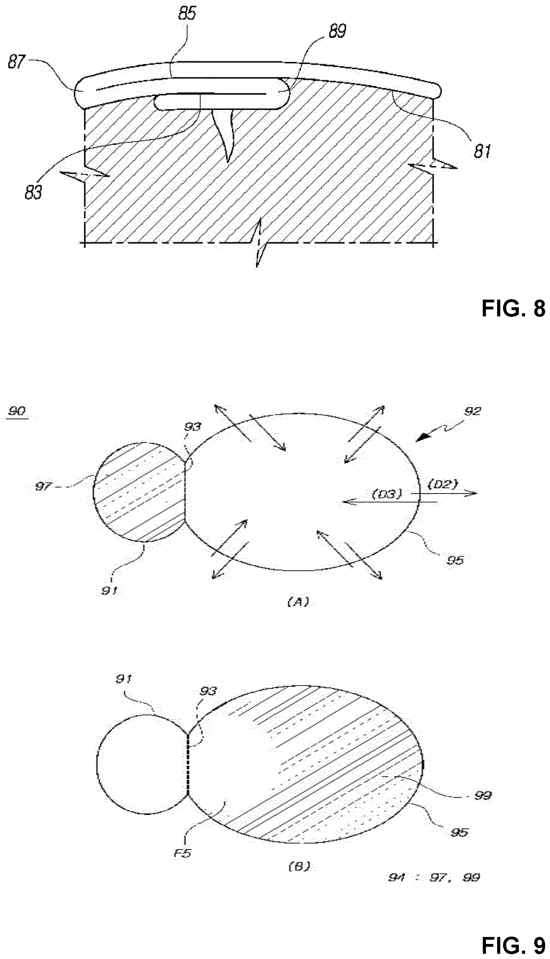

FIGS. 1 and 2 are a perspective view and a side view of a tension attenuating hypertrophic scar reduction film according to a first embodiment of the present invention. FIG. 3 is a plan and a side views of an attenuating hypertrophic scar reduction film according to a second embodiment of the present invention. FIG. 4 is a plan view of a tension attenuating hypertrophic scar reduction film according to a third embodiment of the present invention. FIG. 5 is a view showing a top and a bottom side surfaces of the tension attenuating hypertrophic scar reduction film according to a fourth embodiment of the present invention. FIG. 6 is a side view illustrating a folded state of a tension attenuating hypertrophic scar reduction film of FIG. 5 according to a fourth embodiment of the present invention. FIG. 7 is a top and a bottom side views of an attenuating hypertrophic scar reduction film according to the fifth embodiment of the present invention. FIG. 8 is a side view illustrating a folded state of the tension attenuating hypertrophic scar reduction film of FIG. 7. FIG. 9 is a view showing the top and the bottom sides the tension attenuating hypertrophic scar reduction film according to a sixth embodiment of the present invention.

According to a first embodiment of the present invention, as shown in FIGS. 1 and 2, a tension attenuating hypertrophic scar reduction film may include: a film main body 11 of a synthetic resin; and an adhesive part 13 coated on the film main body 11 so that the film main body 11 is attached to a wound site A, wherein the film main body 11 may be formed in a non-uniform thickness H.

Here, the wound site A may include a stab wound by a knife, for example, during a surgery or a burn site. However, the wound site may be generated by different reason, and is not limited thereto. In addition, in the wound site A, a tension is generated in a direction in which the wound is opened, if the opening of the wound continues due to such tension, the wound is healed and hypertrophic scars are formed at the same time.

Hereinafter, embodiments according to the present invention will be described in detail for attenuating the tension generated in the wound site A to suppress the above-mentioned hypertrophic scar generation.

The film main body 11 is made of a synthetic resin having a predetermined elastic force. The thickness H of the film main body may be formed non-uniformly, such a film main body 11 as the distance away from the wound site A, for example, the thickness H gradually decreases.

In addition, the film main body 11 may be formed symmetrically on both sides with respect to the wound site A so that the both sides of the lengths L1, L2 are the same, more specifically, the film main body 11 may include a variation prevention part 15 configured to be attached the wound site A, and a variable elastic part 17 configured to be extended in both directions from the variation prevention part 15 and formed gradually reduced in thickness H as the distance from the wound site A away.

The variation prevention part 15 is attached to an upper portion of the wound site A, a thickness H of the variation prevention part is thicker than a thickness H of the variable elastic part 17, which is attached to both sides with respect to the wound site A, so that the variation prevention part 15 may not be easily deformed compared to the variable elastic portion 17 by the tension generated at the wound site A.

The variable elastic part 17 is extendedly formed from the variation prevention part 15 and configured to be attached to both sides of the wound site A. The variable elastic part 17 is formed to gradually decrease in thickness as it moves away from the variation preventing part 15, so that the variable elastic part 17 is provided to be easily elastically deformed compared to the variable prevention part 15 by a tension generated at the wound site A.

Therefore, when a tension is generated at the wound site A, the film main body 11, the variable elastic part 17 is formed to have a thinner thickness H than a thickness of the variation prevention portion 15 and is spaced apart from the wound site A at a predetermined interval and attenuates the tension and transmits the attenuated tension to the variation prevention part 15. At this time, the attenuated tension from the variable elastic part 17 is transmitted to the variation prevention part 15, however, an elastic deformation is prevented due to a thicker thickness H of the variation prevention part 15 compared to a thickness of the variable elastic part 17, thereby preventing an opening of the wound site A.

Subsequently, an adhesive part 13 is coated on one side of the film main body 11, i.e., a bottom side surface of the film body 11 contacted with the wound site A is coated so that the film main body 11 is attached to the adhesive part 13.

The adhesive part 13 may be coated on the entire bottom side surface of the film main body 11 to form a coated surface and a plurality of areas may be coated to gradually increase the distance from wound site A, so that the elastic deformation may be made larger as the film body 11 moves away from the wound site A.

On the other hand, a non-uniform tension may be generated on both sides of the wound site A with respect to the wound site A, for example, if a wound occurs in a lower abdomen of a cesarean section laterally, since a tension transmitted from the upper portion of the wound site A is greater than a tension transmitted from the lower portion of wound site A, an upper and a lower asymmetrical tensions are transmitted to the wound site A.

Therefore, if the film main body 11 is symmetric about the wound site A, a strong tension is generated only on one side of both sides of the wound site A due to a strong tension transmitted to the only one side, resulting in scarring due to an opening of the wound.

Thus, as shown in FIGS. 3 and 4, in order to solve the above problems, a tension attenuating hypertrophic scar reduction film may include: a film main body 31, 41 made of a synthetic resin; and an adhesive part 33 coated on the film main body 31, 41 so that the film main body 31, 41 is attached to the wound site A, respectively.

Both sides of the film body 31, 41 are formed with an asymmetry in at least one of length or area with respect to the wound A.

As shown in FIG. 3, lengths of both sides of the film body 31 are formed asymmetric with respect to the wound according to the second embodiment of the present invention.

That is, a tension attenuating hypertrophic scar reduction film 30 may include: a film main body 31 formed in both sides of the length asymmetrically with respect to the wound site A; and an adhesive part 33 coated on one side of the film body 31 so that the film main body 31 is attached to the wound site A according to the second embodiment of the present invention.

The film main body 31 has two different lengths of sides, i.e., a longer side and a shorter side, configured to be attached on both sides of the wound site A, respectively. The longer side of the film main body 31 is attached to one side of the wound site A where a relatively strong tension is generated, and the shorter side of the film main body 31 is attached to the other side of the wound site A where a relatively weaker tension is generated.

An example of the structure of the film main body 31 will be described in more detail. The film main body 31 may include: a longer film part 35 having a length L2 configured to be attached on one side of the wound site A in which a relatively strong tension is generated; and a shorter film part 37 having a length L1 is extended in one direction from the longer film part 35 and the length L1 is shorter than the length L2 of the longer film part 35 is attached to the other side of the wound site A in which a relatively weaker tension is generated.

The adhesive part 33 may be formed on one side surface of the longer film part 35 and the shorter film part 37 such that the longer film part 35 and the shorter film part 37 can be attached to the wound site A, i.e., may be coated on the bottom side of the longer film part 35 and the shorter film part 37 in which the wound site A in contact with.

The adhesive part 33 may be coated on the entire bottom surface of the film main body 31 like the adhesive part 21 of the tension attenuating hypertrophic scar reduction film 10 according to an embodiment of the present invention. A plurality of areas may be coated on the bottom surface of the film main body 31 so as to be spaced apart a predetermined distance along the longitudinal direction of the film main body 31 and the distance is gradually increased as the distance from the wound A increases.

Meanwhile, referring to FIG. 4, a tension attenuating hypertrophic scar reduction film 40 according to the third embodiment of the present invention may include: a film body 41 made of synthetic resin; and an adhesive part (not shown) coated on the film main body 41 so that the film main body 41 is attached to the wound site A, wherein both sides areas of the film main body 41 are formed asymmetrical with respect to the wound site A.

The film main body 41 is formed for having different areas with respect to the wound site A when attached to the wound site A, i.e., a relatively large area of both sides is attached to a side F1 where the tension is relatively strong of the wound site A, and a relatively small of both sides is attached to the other side F2 where the tension is relatively weak area of the wound site A.

With explaining an example of the structure of the film main body 41 in more detail, the film main body 41 may include: a first film part 45 having a predetermined elastic force and configured to be attached to one side F1 of the wound site A where the tension is relatively strong; and a second film part 47 having a predetermined elastic force, configured to be attached to the other side F2 with relatively weak tension in the wound site A and extending in one direction from the first film part 45 and having a smaller area than the area of the first film part 45.

The adhesive part (not shown) is formed on a bottom side surface of the main body 41, which is in contact with the wound part A in the same manner as the adhesive parts 13, 33 of the tension attenuating hypertrophic scar reduction film 10 and 30 according to the first and second embodiments of the present invention.

According to the structure as described above, a tension attenuating hypertrophic scar reduction film 30, 40 may include: a long film body 35, which is relatively long, or a first film body 45, which is relatively large area is attached to one side F1 where relatively strong tension generated at the wound site A, thereby more effectively reducing non-uniform tension generated by the wound site A according to the second and the third embodiments of the present invention.

More specifically, when the tension is generated non-uniformly on the wound site A, in a case the skin is pulled in a direction D1 where the tension is strong, since the elastic force of the long film part 35 and the first film part 45, which is longer than the short film part 37 and the second film part 47 acts in the opposite direction D2 in which the skin is pulled, a tension generated in a direction D1 can be reduced more effectively, thereby suppressing a formation of hypertrophic scar caused by a skin opening.

Meanwhile, referring to FIGS. 5 and 6, a tension attenuating hypertrophic scar reduction film 50 may include: a film main body 53 folded with respect to a folding line 51 and attached to the wound site A; and an adhesive part 55 coated on one or both sides of the film main body 53 so that the film main body 53 is attached to the wound site A according to the fourth embodiment of the present invention.

The film body 53 is attached to the wound site A and folded so that a double surface is formed using the fold line. An example of the structure of the film body 53 will be described in more detail.

The film body 53 may include a first film body 57 attached to the wound site A; and a second film main body 59 extended from an end of the first film main body 57 and folded in a direction in which the first film main body 57 is positioned using the first folding line 51.

The second film main body 59 is extended from the first film main body 57 and is folded in a direction in which the first film main body 57 is positioned with respect to a first folding line 51 to be superimposed with the first film main body 57. If the second film main body 59 is folded through the first folding line 51, the second film main body 59 has a larger length than the first film main body 57 so as to cover the entire first film main body 57.

Subsequently, the adhesive part 55 is coated on one side or both sides of the film body 53 so that the film body 53 is attached to the wound site A, with explaining an example of the adhesive portion 55 in more detail, the adhesive part 55 may include: a first adhesive part 56 coated on one side of both sides of the first film body 57 in contact with the wound site A; and a second adhesive part 58, which is folded with respect to the first folding line 51 of both sides of the second film body 59 and coated on one side of the first film body 57 so as to overlap the first film body 57.

An example of the structure of the adhesive part 55 will be described in more detail. The adhesive part 55 may include: a first adhesive part 56 coated on one side of the first film main body 57 that is in contact with the wound area A; and a second adhesive part 58 coated on one side of the both sides of the second film body 59, which is folded to the first film body 57 with respect to the first folding line 51.

Here, the second adhesive part 58 may be coated on entire one side of a second film main body 59 where folded with respect to the first folding line 51 and overlapped with the first body film 57.

Alternatively, the second film main body 59 may be coated on a remaining area except the area in close contact with the first film main body 57 of one side of the second film main body 59. The non-adhesive part 61 may be formed at a portion where the first film 57 and the second film body 59 are overlapped.

According to this structure, a tension reducing hypertrophic scar reduction film 50 according to an aspect of the fourth embodiment of the present invention, if the second adhesive portion 58 is coated on the entire surface of the second film body 59 in the direction overlapping with the first film body 57, the first film body 57 and the second film body 59 is attached and formed integrally, the thickness thereof is gradually reduced in a direction with respect to the wound site A. In this case, the tension generated non-uniformly in one direction with respect to the wound site A is transmitted to the first and the second film bodies 57 and 59 attenuated by an elastic deformation of the second film body 43. At this time, since the thickness of the film main body 53 to which the first film main body 57 and the second film main body 59 are attached is thicker than that of the second film main body 59, it is possible to prevent the opening of the wound site the deformation does not occur due to the attenuated tension.

On the contrary, in the present invention, the second adhesive part 58 is coated on one side of the second film main body 59 except for the close contact part 54 that comes into close contact with the first film main body 57 so that a nonuniformly generated tension is applied in one direction. Accordingly, a tension is applied in one direction, firstly, the tension is attenuated by the elastic deformation of the second film body 59 and is transmitted to the first film body 57, at this time, since the direction in which the tension is applied is reversed with respect to the bent part 63 is attenuated secondarily, the tension transmitted to the wound site A can be effectively attenuated to prevent the wound from being opened.

Meanwhile, referring to FIGS. 7 and 8, a tension attenuating hypertrophic scar reduction film 70 may include an adhesive part 72, which is coated on one side or both sides of a film part 71 and the film part 71, which are folded in multiple numbers using a fold line according to the fifth embodiment of the present invention.

The film part 71 is folded a plurality of times and attached to the wound site A, with explaining the structure as described above in more detail, the film part 71 may include a first film portion 73 attached to the wound site A; the second film portion 75 which is folded in the direction in which the first film portion 73 is positioned with respect to the first folding line 74 at the end of the first film portion 73 to cover the first film part 73; and a third film part 77.A third that is folded at the end of the second film part 75 in the direction in which the second film portion 75 is located with respect to the second folding line 76 to cover the second film portion 75 and the first film portion 73.

Subsequently, the adhesive part 72 is coated on one side or both sides of the film part 71 so that the film part 71 is attached to the wound site A, with explaining an example of the structure of the adhesive part 72 in more detail, the adhesive part 72 may include: a first adhesive part 78 coated on one side of the first film portion 73 in contact with the wound A; the second adhesive part 79 coated on one side of the second film part 75 in contact with the first film part 75 by folded with respect to the first folding line 74; and the third adhesive part 81 coated on a side of the third film part 77 where in contact with the second film part 75 when folding with respect to the second folding line 76.

Here, the second adhesive part 79 may be applied to and coated on the remaining area except for the area F3 that is in close contact with the first film part 73 of one side of the second film part 75. In this case, the non-adhesive portion 83 is formed at the portion where the film portion 73 and the second film portion 75 overlap.

In addition, the third adhesive part 81 may be applied to and coated on the remaining area except for the region F4 in close contact with the second film part 75 of one side of the third film part 77. The non-adhesive part 85 is formed where the second film part 75 and the third film part 77 overlap.

According to the structure as described above when the tension attenuating hypertrophic scar reduction film 70 according to the fifth embodiment of the present invention is a non-uniform tension occurs when the skin is pulled in one direction, the tension is primarily attenuated by the elastic deformation of the third film part 77, and a direction of the attenuated tension, transmitted from the third film part 77 to the second film part 75, is reversed with respect to the first folded part 87 and the tension secondary attenuated by the reversed direction, a direction of the tension transmitted from the second film portion 75 to the first film portion 73 is reversed with respect to the second folded part 89, and the reversed direction of the tension thirdly attenuated the tension effectively prevent from opening of the wound site.

On the other hand, a tension attenuating hypertrophic scar reduction film 90 according to the sixth embodiment of the present invention may include: a first film part 91 attached to wound site A; a film part 92 includes a second film part 95, wherein the second film part is formed in a substantially circular shape and has a larger area than the first film part 91, and is folded with respect to a folding line 93 in the direction in which the first film part 91 is positioned so as to overlap the first film part 91; and an adhesive part 94, wherein the adhesive part 94 includes a first adhesive part 97 coated on one side of the first film portion 91 in contact with the wound site A, and a second adhesive part 99 coated on one side of the second film part 95 and the first film part 91 overlapped with each other.

Here, the second adhesive part 99 may be applied to and coated on the remaining areas except for the area F5 in close contact with the first film part 91 of one side of the second film part 95.

A tension attenuating hypertrophic scar reduction film 90 according to the sixth embodiment of the present invention by a structure in which the first film 91 and the second film 95 overlap each other while forming a non-adhesive part (not shown) with respect to the folding line 93. The same structure as the tension attenuating hypertrophic scar reduction film 50 can attenuate the tension generated in the wound site A to suppress the hypertrophic scar due to the opening of the wound.

In addition, if a wound occurs laterally in an abdomen during a cesarean section, the tension generated in the wound site A can act all directions D2 due to the shape of the abdomen.

At this time, since the first film 91 is provided in a substantially circular shape so as to correspond to the abdominal shape, effectively providing the elastic force of the film in the direction opposite to the tension direction D3, the tension acting in all directions D2 on the wound site A can be more effectively attenuated a generation of the hypertrophic scar caused by the opening of the wound.

In the above description, it is described that all the components constituting the embodiments of the present invention are combined or operated in one, but the present invention is not necessarily limited to these embodiments. That is, within the scope of the present invention, all of the components may be selectively operated in combination with one or more.

In addition, the terms "comprise", "constitute" or "having" described above mean that the corresponding component may be included unless otherwise stated, and thus, it should be construed that it may further include other components rather than to exclude other components. All terms, including technical and scientific terms, have the same meanings as commonly understood by one of ordinary skill in the art unless otherwise defined. Terms commonly used, such as terms defined in a dictionary, should be interpreted to coincide with the contextual meaning of the related art, and shall not be construed in an ideal or excessively formal sense unless explicitly defined in the present invention.

Although the invention has been discussed in the foregoing with reference to an exemplary embodiment of the processing device of the invention, the invention is not restricted to this particular embodiment which can be varied in many ways without departing from the gist of the invention. The discussed exemplary embodiment shall therefore not be used to construe the appended claims strictly in accordance therewith.

To the contrary, the embodiment is merely intended to explain the wording of the appended claims without intent to limit the claims to this exemplary embodiment. The scope of protection of the invention shall therefore be construed in accordance with the appended claims only, wherein a possible ambiguity in the wording of the claims shall be resolved using this exemplary embodiment. While the present subject matter has been described in detail with respect to specific exemplary embodiments and methods thereof, it will be appreciated that those skilled in the art, upon attaining an understanding of the foregoing may readily produce alterations to, variations of, and equivalents to such embodiments. Accordingly, the scope of the present disclosure is by way of example rather than by way of limitation, and the subject disclosure does not preclude inclusion of such modifications, variations and/or additions to the present subject matter as would be readily apparent to one of ordinary skilled in the art.

## Claims

1. A tension attenuating hypertrophic scar reduction film, the film comprising:
- a film body made of a synthetic resin; and
- an adhesive part coated on the film body so that the film body is attached to a wound site,
wherein a thickness of the film is formed non-uniform.

2. The tension attenuating hypertrophic scar reduction film according to claim 1, wherein the film body is gradually reduced in thickness away from the wound site.

3. A tension attenuating hypertrophic scar reduction film, the film comprising:
- a film body made of a synthetic resin; and
- an adhesive part coated on the film body so that the film body is attached to a wound site,
wherein any one or more of the length or area of both sides of the film body is formed asymmetrically with respect to the wound site.

4. The tension attenuating hypertrophic scar reduction film of according to claim 3, wherein the lengths of both sides of the film body are asymmetrically formed with respect to the wound site and the one side of the relatively long length of the two sides is attached to one side of the relatively high tension of the wound site and the other side region having a relatively short length of the both sides is attached to the other side of which the tension is relatively weak at the wound site.

5. The tension attenuating hypertrophic scar reduction film according to claim 3, wherein the film body is formed asymmetrical with respect to the wound site, a relatively large area side of the film is attached to one side of which a tension is relatively strong side of the wound site and a relatively small area side of the film is attached to one side of which a tension is relatively weak side of the wound site.

6. A tension attenuating hypertrophic scar reduction film, the film comprising:
- a film main body is folded using a fold line and attached to a wound site; and
- an adhesive part coated on one side or both sides of the film body so that the film body is attached to the wound site.

7. The tension attenuating hypertrophic scar reduction film according to claim 6, wherein the film main body comprises:
- a first film main body attached to the wound site; and
- a second film main body extended from an end of the first film main body and folded in a direction in which the first film main body is positioned using a first folding line to overlap the first film main body.

8. The tension attenuating hypertrophic scar reduction film according to claim 7, wherein the adhesive part includes: a first adhesive part coated on one side of the first film body where the wound part in contact with; and a second adhesive part is coated on one side of the second film main body where overlapped side with the first film main body when folded with respect to the first folding line.

9. The tension attenuating hypertrophic scar reduction film of claim 8, wherein the second adhesive part is coated on a remaining part of the one side of the second film body except the area in close contact with the first film body.
